# EUROPEAN PATENT APPLICATION

(11) **EP 3 772 291 A1**
(43) Date of publication of application: **10.02.2021**
(21) Application number: 19191059.5
(22) Date of filing: 09.08.2019
(51) Int. Cl.: A41D 13/06, A41D 13/08, A61F 13/02, A41D 13/05

(54) **SELF-ADHESIVE SUN PROTECTION DRESSING TO BE PLACED ON A BODY PART**

(71) Applicant: Donoso, Elena Pérez, 08011 Barcelona (ES); Breitenbach, Siegfried, 97859 Wiesthal (DE)
(72) Inventor: Donoso, Elena Pérez, 08011 Barcelona (ES)
(74) Representative: Gleim, Christian Ragnar

(57) **Abstract**

Self-adhesive sun protection dressing (1) of a size and shape to be placed and fit on a body part (2,3) comprising a textile layer (4) with a UV protection factor of at least 40, with a top side (4a) and a bottom side (4b), wherein an adhesive layer (5) being arranged at the bottom side (4b).

## Description

This invention relates to the field of sun protection for covering and protecting parts of the human body.

Body part dressings are already known to be bandages, sticking plaster or plaster of all types.

In the field of sun protection products, they are known to have the ability to screen or block out the sun's burning rays on body parts when exposed to the sun, for example by use of gloves and/or glove like products, shoes and/or shoe like products for hand/foot coverage. However, the backside of the human hand as well as the instep or back of the foot are undressed and uncovered hence these parts are constantly exposed to damaging sunrays.

A disadvantage related to the full covering apparel currently found in the market is that it is inconvenient for the user due to their lack of comfort, their indiscreet appearance, and/or lack in practicality when the user only wishes to cover parts of the hand, such as palm and fingers, free of coverage or of coverage material/garment. Furthermore, garments currently available in the market for covering the hand are primarily used to cover the hand entirely, and when not so, they do use some attachment to fingers thus limiting the natural free maneuverability of the entire hand, its agility and general sense of tact.

The market currently does not address the need to cover and protect specific body parts from UV rays while allowing the entire hand and foot to have full range of movement and sense of complete bareness and no coverage elsewhere providing the user complete freedom with regard to the coverage.

Hence, an unmet need exists to create an adhesive, SPF, water resistant and disposable garment/plaster that can be latched to the backside of the human hand and to the instep of the foot at any given moment to shield these body parts from direct exposure to the sun while allowing the user a free maneuverability of the remaining parts of the hand and foot, all with a discreet appearance. The present invention seeks to provide this unmet need. It is important to state explicitly, however, that this invention may have other applications.

The present invention comprises an self-adhesive sun protection dressing (element) of a size and shape to be placed and fit on a body part comprising a textile layer with a UV protection factor of at least 40 (in particular in the range of 45 to 55), with a top side and a bottom side. The adhesive layer is arranged at the bottom side. The UV protection factor (SPF - sun protection factor) can be determined for clothing according to New Zealand Standard 4399:1996 for sun protecting clothing evaluation and classification (AS/NZS4399: 1996).

The self-adhesive sun protection dressing to be placed and fit on the dorsal surface of the hand is cut in a shape that covers the area from the wrist to the base of the fingers and from one side of the hand to the other side of the hand, thus covering the entire dorsal of the hand. In that way the sun protection dressing can be of a size and shape to resemble a body part, i.e. the dorsal surface of the hand. In an alternative the sun protection dressing can be of a size and shape which comprises a circular, square and or rectangular geometrical shape.

According to a preferred aspect, the textile layer has a size and shape to be placed on the dorsal surface of the hand (backside of the hand) or instep (backside) of the foot (independent from each other). The textile layer has an asymmetrical shape either corresponding to the left or right dorsal surface of the hand (backside of the hand), or either corresponding to the left or right instep of the foot. Preferred is a self-adhesive sun protection dressing, wherein the textile layer has an asymmetrical shape either corresponding to the left or right dorsal surface of the hand (backside of the hand), or corresponding either to the left or right instep of the foot. The asymmetric shape adapted to the left or right back of the hand or the left or right instep of the foot improves the fitting of the self-adhesive sun protection dressing.

It is particularly preferred if the textile layer is of a water repellent material or has a water repellent coating. A water-repellent textile layer contributes to the self-adhesive sun protection dressing by allowing for sticking to the skin for a longer period of time while the person is in the water.

It is of advantage if at least the top surface of the textile layer has a color corresponding to a skin color. This avoids an optically indiscreet appearance while wearing. However this invention includes textile corresponding to any color or design.

It is particularly preferred if the textile layer has a density between 130 g/m² and 200 g/m². The textile layer is therefore light enough to be worn without disturbing influence for example during sporting activities.

It is particularly preferred if the adhesive layer comprises acrylic adhesive. Acrylic adhesive is particularly preferred due to its high resistance to UV light and moisture.

Advantageously, the adhesive is chosen with an adhesive strength of the self-adhesive sun protection dressing on human skin is between 1 N/cm and 6 N/cm. The self-adhesive sun protection dressing is thus able to adhere to the skin for a particularly long time without undesired detachment.

Preferably, the textile layer comprises an elastomeric (polyether-polyurea copolymer) fiber material. An elastomer fiber contributes advantageously to the wearing comfort as it can adapt to the movement of the hand and foot through deformation.

It is particularly preferred if the elastomeric fiber is elastane (Spandex).

According to another advantageous aspect, the textile layer comprises nylon and/or viscose and/or cotton and/or polyester fiber. These fibers are particularly skin-friendly.

A clearer understanding of the key features of the invention summarized above may be achieved by reference to the appended drawings in which
- Fig. 1: is an illustration of the self-adhesive sun protection dressing according to a first embodiment of the invention to be attached on the dorsal or backside of the left and right hand;
- Fig. 2: illustration of the self-adhesive sun protection dressing cut in a shape that resembles the general shape of the instep of the human left and right foot including the area from the ankle to the base of the toes and from one side of the instep to the other side of the instep; and
- Fig. 3: schematic illustration of a cross section of a part of the self-adhesive sun protection dressing.

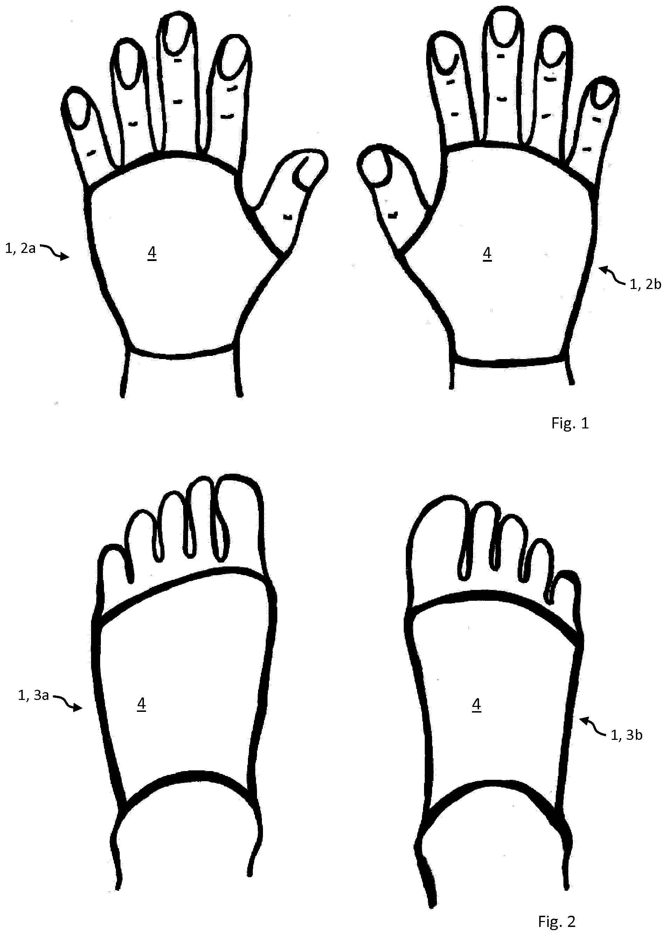

**Fig. 1** shows an illustration of the self-adhesive sun protection dressing 1 cut in a shape that resembles the general shape of the dorsal part of the human left and right hand 2a, 2b including the area from the wrist to the base of the fingers and from one side of the hand to the other side of the hand, thus covering the entire dorsal of the left or right hand 2a, 2b. This illustration shows the cover as it would appear on the backside of the left or right hand 2a, 2b.

Moreover, the textile layer 4 has an asymmetrical shape corresponding to the left or right dorsal surface of the hand 2a, 2b. The asymmetric shape adapted to the left or right back of the hand 2a, 2b improves the fitting of the self-adhesive sun protection dressing 1. Said textile layer 4 comprises elastane as elastomeric fiber. The elastomeric fiber contributes advantageously to the wearing comfort as it can adapt to the movement of the hand through deformation. Furthermore the textile layer 4 comprises polyester fiber, which is a particularly skin-friendly fiber. The exact materials used to manufacture this invention in any of its parts as well as the shape of each and all-individual parts can vary from design to design.

**Fig. 2** shows an illustration of the self-adhesive sun protection dressing 1 cut in a shape that resembles the general shape of the instep of the left and right human foot 3a, 3b including the area from the ankle to the base of the toes and from one side of the instep to the other side of the instep, thus covering the entire instep of the foot 3a, 3b. This illustration shows the cover as it would appear on the instep of the left and right foot 3a, 3b.

Moreover, the textile layer 4 has an asymmetrical shape corresponding to the left or right instep of the foot 3a, 3b. The asymmetric shape adapted to the left or right instep of the foot 3a, 3b improves the fitting of the self-adhesive sun protection dressing 1. Said textile layer 4 comprises elastane as elastomeric fiber. The elastomeric fiber contributes advantageously to the wearing comfort as it can adapt to the movement of the foot through deformation. Furthermore the textile layer 4 comprises polyester fiber, which is a particularly skin-friendly fiber. The exact materials used to manufacture this invention in any of its parts as well as the shape of each and all-individual parts can vary from design to design.

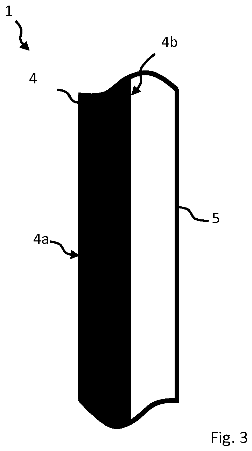

**Fig. 3** shows a schematic cross section of a part of the self-adhesive sun protection dressing 1 comprising a textile layer 4 with a UV protection factor of at least 40, with a top side 4a and a bottom side 4b. Particularly the textile layer 4 shown is water repellent and has a density of 130 g/m². The top surface 4a of the textile layer 4 has a color corresponding to a skin color. This avoids indiscreet appearance while wearing. An adhesive layer 5 is arranged at the bottom side 4b. The adhesive layer 5 comprises acrylic adhesive with an adhesive strength between 1 N/cm and 6 N/cm on human skin. Acrylic adhesive is particularly preferred due to its high resistance to UV light and moisture.

## Claims

1. Self-adhesive sun protection dressing (1) of a size and shape to be placed and fit on a body part (2, 3) comprising a textile layer (4) with a UV protection factor of at least 40, with a top side (4a) and a bottom side (4b), wherein an adhesive layer being arranged at the bottom side (4b).

2. Self-adhesive sun protection dressing (1) according to claim 1, wherein the textile layer (4) has a size and shape to be placed the dorsal surface of the hand (2) or instep of the foot (3) (2a, 2b), or either corresponding to the left or right instep of the foot (3a, 3b).

3. Self-adhesive sun protection dressing (1) according to claim 1 or 2, wherein the textile layer (4) is of a water repellent material or has a water repellent coating.

4. Self-adhesive sun protection dressing (1) according to any one of the preceding claims, wherein at least the top surface (4a) of the textile layer (4) has a color corresponding to a skin color.

5. Self-adhesive sun protection dressing (1) according to any one of the preceding claims, wherein the textile layer (4) has a density between 130 g/m² and 200 g/m².

6. Self-adhesive sun protection dressing (1) according to any one of the preceding claims, wherein the adhesive layer (5) comprises acrylic adhesive.

7. Self-adhesive sun protection dressing (1) according to any one of the preceding claims, wherein the adhesive strength of the self-adhesive sun protection dressing (1) on human skin is between 1 N/cm and 6 N/cm.

8. Self-adhesive sun protection dressing (1) according to any one of the preceding claims, wherein the textile layer (4) comprises an elastomeric fiber material.

9. Self-adhesive sun protection dressing (1) according to claim 8, wherein the elastomeric fiber is elastane.

10. Self-adhesive sun protection dressing (1) according to any one of the preceding claims, wherein the textile layer (4) comprises nylon and/or viscose and/or cotton and/or polyester fiber.
